# EUROPÄISCHE PATENTANMELDUNG

(11) **EP 4 520 417 A1**
(43) Veröffentlichungstag der Anmeldung: **12.03.2025**
(21) Anmeldenummer: 23196385.1
(22) Anmeldetag: 08.09.2023
(51) Int. Cl.: B01D 15/18, A61K 31/00, C07D 311/80

(54) **VERFAHREN ZUM HERSTELLEN VON CANNABINOIDEN, INSBESONDERE DRONABINOL MITTELS VERTEILUNGSCHROMATOGRAPHIE**

(71) Anmelder: Avextra Innovations GmbH, 64625 Bensheim (DE)
(72) Erfinder: BONNLÄNDER, Bernd, 90571 Schwaig (DE); FERTIG, Robin, 64287 Darmstadt (DE)
(74) Vertreter: Simandi, Claus

(57) **Zusammenfassung**

Die Erfindung betrifft ein Verfahren zum Herstellen von Cannabinoiden, insbesondere Dronabinol und CBD, dessen Isolierung mittels *Centrifugal Partition Chromatography* (kurz: CPC) aus einem Cannabisextrakt.

## Beschreibung

Die Erfindung betrifft ein Verfahren zum Herstellen von Cannabinoiden, insbesondere Dronabinol und CBD, dessen Isolierung mittels *Centrifugal Partition Chromatography* (kurz: CPC) aus einem Cannabisextrakt.

Die CPC wird zur Gewinnung und Anreicherung von Pflanzeninhaltsstoffen aus Pflanzenextrakten im analytischen, semipräparativen sowie präparativen Maßstab eingesetzt. Die CPC ist ein flüssig-flüssig Chromatographieverfahren unter Verwendung eines mindestens zweiphasigen Lösungsmittelsystems, wobei eine erste Phase durch Zentrifugalkraft stationär gehalten wird und eine zweite nicht mischbare flüssige Phase.

Die CPC ermöglicht eine nahezu verlustfreie Separation hochkomplexer Substanzgemische aus Rohextrakten. Hersteller solcher Zentrifugal-Verteilung-Chromatographen zur Durchführung einer CPC ist z.B. Gilson Incorporate (Middleton, WI, USA) oder RotaChrom Technologies LLC (Budapest, Ungarn).

Bei der CPC-Methode wird, wie bei gängigen flüssig-flüssigchromatographischen Methoden, wie zum Beispiel der High Speed Countercurrent Chromatography (HSCCC), ein 2-phasiges Lösungsmittelgemisch eingesetzt. Es kann wahlweise die obere oder die untere Phase als stationäre Phase verwendet werden. Anders jedoch als bei der HSCCC wird bei der CPC nicht mit einer Kapillarspule, sondern mit einem mit mehreren hundert Trennkammern versehenen Rotor gearbeitet. In diesen direkt hintereinander geschalteten Kammern findet die Verteilung der im Pflanzenextrakt enthaltenen Substanzen zwischen mobiler und stationärer Phase statt.

Das System wird während der Trennung in schnelle Rotation versetzt (bis zu 2.500 rpm). Je nach Durchflußrichtung wird dadurch zum einen die gewünschte Phase im Rotor der CPC zurückgehalten, und zum anderen die Trennung der beiden Phasen durch die Zentrifugalkraft beschleunigt. Dies ermöglicht die Verwendung großer Flußgeschwindigkeiten und folglich den Durchsatz großer Substanzmengen in kurzer Zeit, so dass eine präparative Anwendung dieser Trenntechnik möglich ist.

Im Stand der Technik beschreiben Hazekamp, A., 2007, Doctoral thesis, Leiden University und Arno Hazekamp, Ruud Simons, Anja Peltenburg-Looman, Melvin Sengers, Rianne van Zweden, Robert Verpoorte, Preparative isolation of cannabinoids from Cannabis sativa by centrifugal partition chromatography, J. Liq. Chrom. Rel. Technol. 2004, 27(15): 2421-2439, die präparative Gewinnung von Cannabinoiden mittels CPC.

Weiterhin ist im Stand der Technik beschrieben, dass Cannabinoide mittels CPC erhalten werden können (WO2016135346A1).

Dennoch werden die Cannabinoide, wie z.B. THC (A, "Dronabinol") oder CBD (B, Cannabidiol) nicht in absoluter Reinheit erhalten.

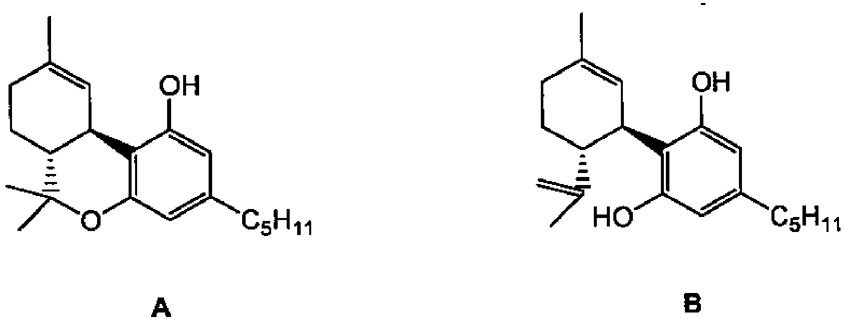

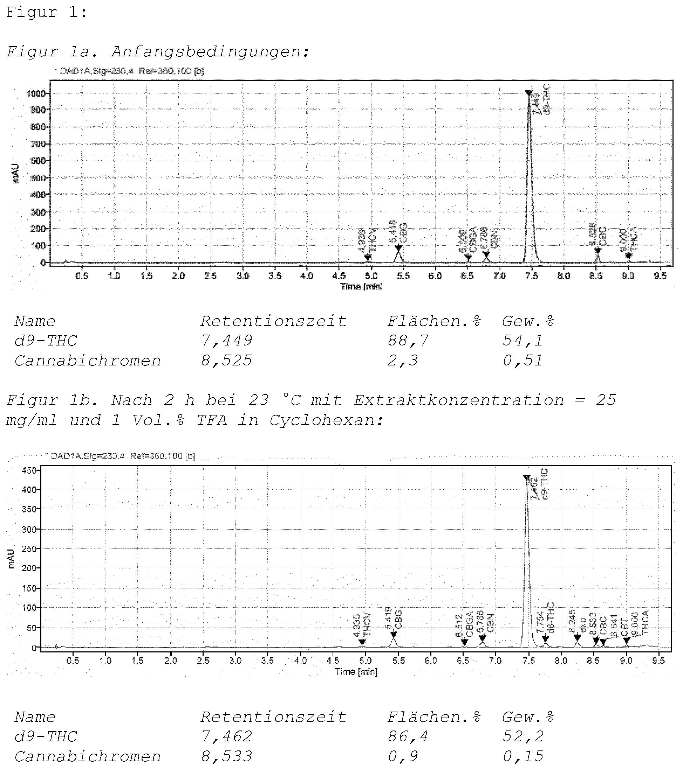

Abb. 1: Dronabinol (A) ((6aR, 10aR)-6a,7,8,10a-tetrahydro-6,6,9-trimethyl-3-pentyl-6H-dibenzo[b,d]pyran-1-ol, Δ⁹-Tetrahydrocannabinol (Δ⁹THC)), Cannabidiol (CBD) (B) (2-[(1R,6R)-3-Methyl-6-prop-1-en-2-yl-1-cyclohex-2-enyl]-5-pentylbenzo-1,3-diol)

Dronabinol ist in Deutschland und anderen Staaten als verschreibungspflichtiges Betäubungsmittel für die Herstellung von Rezepturarzneimitteln in Apotheken erhältlich. In den USA ist es unter dem Handelsnamen Marinol^{®} zur Behandlung von Anorexie und Kachexie bei AIDS und als Antiemetikum im Rahmen einer Krebstherapie zugelassen. Weiterhin ist die Therapie eines zu hohen Augeninnendruckes (Glaukom) eine geeignete Indikation für Dronabinol.

Die chromatographische Trennung und präparative Aufreinigung und Isolation von Cannabinoiden stellt nach wie vor eine Herausforderung dar, insbesondere die Gewinnung von Cannabinoiden, vorzugsweise Δ⁹THC und CBD in hoher Reinheit von mehr als 96 %.

Insbesondere ist Cannabichromen (CBC-Cs) ein hochanteiliger Bestandteil, welcher bei der Durchführung einer CPC zum Herstellen und Isolieren von Dronabinol oder CBD schwer oder nicht abtrennbar ist und folglich als unerwünschte Verunreinigung bleibt.

Es stellt sich daher die Aufgabe, ein verbessertes Verfahren für die Trennung und/oder Isolation von Cannabinoiden, insbesondere Dronabinol oder CBD, aus Cannabis Pflanzenextrakten bereitzustellen, welches Cannabichromen selektiv entfernt oder zumindest reduziert, und insbesondere in der Lage ist, eine höhere Ausbeute und Reinheit an Cannabinoiden, insbesondere CBD und / oder THC, vorzugsweise Dronabinol (Δ⁹THC) präparativ bereitzustellen, und zwar im Rahmen einer Flüssig-flüssig-Verteilungschromatographie (CPC).

Die Aufgabe wird durch ein Verfahren nach einem der Patentansprüche gelöst.

Das erfindungsgemäße Verfahren erlaubt vorteilhaft die quantitative und selektive Umlagerung von Cannabichromen in einem Cannabisextrakt, so dass Cannabichromen zumindest reduziert oder vollständig entfernt wird. In einem derart behandelten Extrakt, welcher zur CPC eingesetzt wird, verbleibt ein Restgehalt an Cannabichromen von 0,0, insbesondere 0,5 - 2 Flächenprozent (siehe HPLC-Chromatogramm gemäß Figur 1).

Demgemäß betrifft die Erfindung ein Verfahren zum Herstellen von Cannabinoiden aus einem Cannabis-Extrakt, aufweisend folgende Schritte:
i.) Trocknen eines Cannabis Extrakts,
ii.) Aufnehmen von i.) in mindestens einem unpolaren aprotischen Lösungsmittel enthaltend mindestens eine protische Säure, wobei das Verhältnis von protischer Säure zum Extrakt im unpolaren aprotischen Lösungsmittel 0,1 - 3,0 Vol % : 5 - 250 mg/ml beträgt, vorzugsweise 0,1 - 1,0 Vol % : 15 - 100 mg/ml,
iii.) Durchführen einer Flüssig-flüssig-Verteilungschromatographie von ii.) unter Verwendung eines mindestens zweiphasigen Lösungsmittelsystems, wobei eine erste Phase durch Zentrifugalkraft stationär gehalten wird und eine zweite nicht mischbare flüssige Phase,
iv.) Isolieren der Cannabinoide aus iii.).

Das erfindungsgemäße Verfahren erlaubt eine Isolation von Cannabinoiden, vorzugsweise Δ⁹THC (INN: Dronabinol) und CBD in hoher Reinheit und Ausbeute von mehr als 96 oder gar 99 %. Insbesondere wird gemäß Schritt ii.) die Reaktion der Umlagerung von Cannabichromen in andere Cannabinoide ermöglicht, so dass diese Umlagerungsprodukte sicher durch die CPC voneinander getrennt werden können.

Besonders vorteilhaft wird Cannabichromen zumindest reduziert oder vollständig aus dem Extrakt entfernt, sodass eine verbesserte Trennung der Cannabinoide im CPC-Verfahren erfolgen kann.

In einer bevorzugten Ausführungsform des erfindungsgemäßen Verfahrens zum Herstellen von Cannabinoiden aus einem Cannabis-Extrakt, weist das mindestens eine unpolare aprotische Lösungsmittel gemäß ii.) eine Dielektrizitätskonstante ε bis 15, insbesondere bis 10 bei Normalbedingungen auf, insbesondere solche wie Alkane, Cycloalkane, Hexan, Heptan, Octan, Cyclohexan, Cycloheptan.

Die Dielektrizitätskonstante ε (bzw. relative Permittivität, wobei die Referenz Vakuum als 1 gesetzt wird) eines Lösungsmittels ist ein Maß für die Fähigkeit des Lösungsmittels, elektrische Felder zu polarisieren und somit die Kapazität eines Kondensators zu beeinflussen, wenn das Lösungsmittel als Dielektrikum zwischen den Kondensatorplatten verwendet wird, und zwar in Abhängigkeit der Polarität, Dipolmoment und Molekülmasse des Lösungsmittels.

In einer bevorzugten Ausführungsform des erfindungsgemäßen Verfahrens zum Herstellen von Cannabinoiden aus einem Cannabis-Extrakt, ist die protische Säure gemäß ii.) ausgewählt aus ein oder mehreren Mineralsäuren, organischen Säuren, insbesondere Salzsäure, Schwefelsäure, Phosphorsäure, Ameisensäure, Essigsäure oder deren halogenierten Varianten wie Trichlor- oder Trifluoressigsäure.

Im Sinne dieser Erfindung ist eine protische Säure als Protonendonator in der Lage, Protonen (H⁺-Ionen) abzugeben. Die protische Säure kann daher in wässrigen Lösungen oder anderen Lösungsmitteln Protonen an Basen (Protonenakzeptor) übertragen.

In einer bevorzugten Ausführungsform des erfindungsgemäßen Verfahrens zum Herstellen von Cannabinoiden aus einem Cannabis-Extrakt, wird Schritt ii.) bei einer Temperatur von 15 - 70, insbesondere 20 - 50 Grad Celsius ausgeführt, und optional je höher die Volumenkonzentration im Verhältnis gewählt wird, desto niedriger kann die Temperatur gewählt sein.

In einer bevorzugten Ausführungsform des erfindungsgemäßen Verfahrens zum Herstellen von Cannabinoiden aus einem Cannabis-Extrakt, beträgt in Schritt ii.) die Reaktionszeit 0,1h - 4h insbesondere 0,5h - 3h, und optional je höher die Temperatur gewählt wird, desto niedriger kann die Reaktionszeit gewählt sein.

In einer weiteren Ausführungsform der Erfindung kann ein Stoppen der Reaktion gemäß Schritt ii.) beliebig erfolgen, insbesondere kann durch einen zusätzlichen Schritt ii*) und zwar Zugabe eines protischen Lösungsmittels die Reaktion gemäß Schritt ii.) beendet werden.

In einer bevorzugten Ausführungsform des erfindungsgemäßen Verfahrens zum Herstellen von Cannabinoiden aus einem Cannabis-Extrakt, weist das protische Lösungsmittel gemäß ii*.) eine Dielektrizitätskonstante ε mehr als 15, insbesondere mehr als 20 bei Normalbedingungen auf, insbesondere Alkohole, Methanol, Ethanol, iso und n-Propanol u.a..

Daher betrifft die Erfindung in einer bevorzugten Ausführungsform ein Verfahren zum Herstellen von Cannabinoiden aus einem Cannabis-Extrakt, aufweisend folgende Schritte:
i.) Trocknen eines Cannabis Extrakts,
ii.) Aufnehmen von i.) in mindestens einem unpolaren aprotischen Lösungsmittel enthaltend mindestens eine protische Säure, wobei das Verhältnis von protischer Säure zum Extrakt im unpolaren aprotischen Lösungsmittel 0,1 - 1,0 Vol % : 15 - 100 mg/ml beträgt,
ii*.) Zugabe eines protischen Lösungsmittels,
iii.) Durchführen einer Flüssig-flüssig-Verteilungschromatographie von ii*.) unter Verwendung eines mindestens zweiphasigen Lösungsmittelsystems, wobei eine erste Phase durch Zentrifugalkraft stationär gehalten wird und eine zweite nicht mischbare flüssige Phase,
iv.) Isolieren der Cannabinoide aus iii.).

Die erfindungsgemäße eingesetzte Centrifugal Partition Chromatography (CPC), d.h. eine Verteilungschromatographie unter Verwendung eines mindestens zweiphasigen Lösungsmittelsystems zeigen vorteilhaft einen geringen Eluentenverbrauch neben keinen Ablagerungen an einer stationären Phase, so dass keine Regeneration der stationären Phase, bzw. Aufbereitung oder Entfernung von störenden Komponenten vonnöten ist. Die Produktwiederfindung ist nahezu quantitativ, da stationäre und mobile Phase einfach vertauscht werden können. Zudem wird die stationäre Phase bei jedem Lauf vollständig erneuert und kann durch Destillation einfach aufgereinigt bzw. erneuert werden. Die Reinheit der isolierten Cannabinoide ist oftmals so hoch, dass weitere chromatographische Aufreinigungsschritte entfallen können. Das Verfahren ist ebenfalls im großtechnischen Maßstab durchführbar.

In einer weiteren vorteilhaften Ausführungsform ist für Cannabinoide die vorteilhafte Verwendung der beiden nicht miteinander mischbaren Lösungsmittel insbesondere Methanol und Cyclohexan bei einem Fluß von 50 bis 3000 mL pro min. bevorzugt, vorzugsweise bei einem Fluss von 200 bis 300 mL pro min. während der Trennung und max. Fluss beim Spülen, bei einer Umdrehungszahl von 50 bis 1.500 rpm, vorzugsweise einer Umdrehungszahl von 900 bis 1.100 rpm während der Trennung bzw. Isolation der Cannabinoide.

In einer weiteren Ausführungsform der Erfindung ist die erste oder zweite Phase gemäß iv.) ausgewählt aus der Gruppe n-Hexan, n-Heptan, Cyclohexan, Methanol, n- oder iso-Propanol, Chloroform, Ethylacetat, Butylmethylether, Acetonitril, Ethanol, Wasser, n-Butanol, Dichlormethan, Tetrahydrofuran, Isooctan, insbesondere eine Mischung davon. Der Fachmann ist in der Lage geeignete Phasen bereitzustellen.

"Cannabis Extrakt" im Sinne dieser Erfindung bedeutet ein beliebig aufgearbeiteter Extrakt aus einer Cannabispflanze oder Hanfpflanze, welche Cannabinoide enthält. Der Extrakt kann ein Primärextrakt, oder teilaufgearbeiteter Extrakt sein. Die Herstellung von Cannabis Extrakten ist im Stand der Technik hinreichend beschrieben.

Besonders vorteilhaft kann die Gewinnung von Cannabinoiden aus beliebigen Cannabispflanzen und deren Extrakte erfolgen. Dies umfasst erfindungsgemäß solche Cannabis Extrakte aus Cannabispflanzen (*Cannabis sativa, Cannabis indica, Cannabis ruderalis*)*,* wie Hanf, Industriehanf, Nutzhanf, Drogenhanf, Faserhanf u.a.. Die bevorzugten Extrakte umfassen wässrigethanolische Auszüge, ölige Auszüge oder Extrakte mittels superkritischer (SFC) oder verflüssigter Gase wie Kohlendioxid (CO₂), Propan oder Butan. Als Extraktionsmittel kommen Wasser, Methanol, Ethanol, Hexan, Heptan, Kohlendioxid o.a. zum Einsatz, auch in Mischungen davon oder mit flüssigen Gasen.

Im Rahmen dieser Erfindung, werden unter Cannabinoiden insbesondere die folgenden Stoffe verstanden:
*Cannabigerol-artige (CBG):* Cannabigerol ((E)-CBG-C₅), Cannabigerol Monomethylether ((E)-CBGM-C₅), Cannabinerolsäure ((Z)-CBGA-C₅), Cannabigerovarin ((E)-CBGV-C₃), Cannabigerolsäure ((E)-CBGA-C₅), Cannabigerolsäure A/B Monomethylether ((E)-CBGAM-C₅ A und/oder B), Cannabigerovarinsäure ((E)-CBGVA-C₃);
*Cannabichromen-artige (CBC):* Cannabichromen (CBC-C₅), Cannabichromensäure A/B (CBCA-C₅ A und/oder B), Cannabichromevarin (CBCV-C₃), Cannabichromevarinsäure A/B (CBCVA-C3 A und/oder B);
*Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃);
*Cannabinodiol-artige (CBND):* Cannabinodiol (CBND-C₅), Cannabinodivarin (CBND-C₃);
*Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A/B (Δ9-THCVA-C₃ A und/oder B), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅), (-)-Δ8-trans-(6aR,10aR)-Tetrahydrocannabinolsäure A/B (Δ8-THCA-C₅ A und/oder B); (-)-(6aS,10aR)-Δ9-Tetrahydrocannabinol ((-)-cis-Δ9-THC-C₅);
*Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A/B (CBNA-C₅ A oder B), Cannabinolmethylether (CBNM-C₅)
*Cannabitriol-artige (CBT):* (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((±)-cis-CBT-C₅), (-)-(9R,10R)-trans[10-O-Ethyl-cannabitriol] ((-)-trans-CBT-OEt-C₅), (±)-(9R,10R/9S,10S)-Cannabitriol-C₃ ((±)-trans-CBT-C₃),8,9-Dihydroxy-Δ6a(10a) tetrahydrocannabinol (8,9-Di-OH-CBT-C₅), Cannabidiolsäure (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
*Cannabielsoin-artige (CBE):* (5aS,6S,9R,9aR)- C₅-Cannabielsoin (CBE-Cs), (5aS,6S,9R,9aR)-C₃-Cannabielsoin (CBE-C₃), (5aS,6S,9R,9aR)-Cannabielsoinsäure A/B (CBEA-Cs A und/oder B), (5aS,6S,9R,9aR)-C3-Cannabielsoinsäure A/B (CBEA-C₃ A und/oder B), Cannabiglendol-C3 (OH-iso-HHCV-C₃), Dehydrocannabifuran (DCBF-C₅), Cannabifuran (CBF-C₅);
*Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-Δ7-trans-(1R,3R,6R)-Isotetrahydrocannabivarin;
*Cannabicyclol-artige (CBL):* (+)-(1aS,3aR,8bR,8cR)-Cannabicyclol (CBL-C₅), (±)-(1aS,3aR,8bR,8cR)-Cannabicyclolsäure A/B (CBLA-Cs A und/oder B), (±)-(1aS,3aR,8bR,8cR)-Cannabicyclovarin (CBLV-C₃);
*Cannabicitran-artige (CBT):* Cannabicitran (CBT-C₅);
*Cannabichromanon-artige (CBCN):* Cannabichromanon (CBCN-C₅), Cannabichromanon-C3 (CBCN-C₃), Cannabicoumaronon (CBCON-C₅).
Besonders bevorzugt sind jedoch erfindungsgemäß Cannabidiol (CBD-C₅) und Δ9-Tetrahydrocannabinol (Δ9-THC-C₅).

Unter dem Begriff "Flüssig-flüssig-Verteilungschromatographieschritt unter Verwendung eines mindestens zweiphasigen Lösungsmittelsystems, wobei eine erste Phase durch Zentrifugalkraft stationär gehalten wird und eine zweite nicht mischbare flüssige Phase" im Sinne der vorliegenden Erfindung wird insbesondere eine Chromatographie verstanden, bei der wie folgt vorgegangen wird:

Eine bestimmte Menge eines Substanzgemisches aus iv.) wird mit einer flüssigen mobilen Phase durch eine stationär gehaltene Phase geleitet, wobei diese durch eine Zentrifugalkraft stationär gehalten wird. In einer weiteren bevorzugten Ausführungsform kann die Flüssig-flüssig-Verteilungschromatographie kontinuierlich durchgeführt werden. Hierzu werden die beiden Phasen im Gegenstrom geführt und eine kontinuierliche Abtrennung statt eines zeitversetzten (diskontinuierlichen) Austritts wird erreicht (siehe z.B. Yin, Lianhong; Li, Yingnan; Lu, Binan; Jia, Yujie; Peng, Jinyong, Trends in Counter-Current Chromatography: Applications to Natural Products Purification Separation and Purification Reviews (2010), 39(1-2), 33-62).

Entsprechend ihrer unterschiedlich starken Wechselwirkungen mit der stationären Phase treten die Substanzen kontinuierlich oder diskontinuierlich aus und können getrennt werden. Während jedoch bei der Flüssigchromatographie die stationäre Phase aus einem gepackten Festbett in einer Säule besteht, handelt es sich bei der Flüssig-flüssig-Verteilungschromatographie um eine zweite nicht mischbare flüssige Phase, die durch geeignete Vorrichtungen, wie z. B. einen Rotor, mittels Zentrifugalkraft stationär gehalten wird, insbesondere mittels einem entsprechenden Zentrifugal-Verteilung Chromatograph (supra).

Diese Beispiele und Figuren dienen ausschließlich zur Erläuterung der Erfindung, ohne die Erfindung auf diese Beispiele zu beschränken.

Figur 1 zeigt die Reduktion von CBC-C₅ durch das erfindungsgemäße Verfahren. Der Hauptbestandteil von CBC ist Cannabichromen (CBC-C₅).

### Beispiele Lösungsmittel:

Verschiedene aprotische und protische organische Lösungsmittel wurden verwendet, um Cannabisextrakt (30mg/ml) zu lösen und das enthaltene CBC-C₅ zu reduzieren bzw. zu entfernen.

| Nr. | Lösungsmittel | Flächenprozent CBC-C₅ nach 2h | Bewertung |
|---|---|---|---|
| 1 | Methanol | 2,3 | - |
| 2 | Cyclohexan | 1, 1 | +++ |
| 3 | Heptan | 1,5 | + |
| 4 | Ethanol | 2,1 | - |
| 5 | Hexan | 1,3 | ++ |

### Beispiele Säuren:

Verschiedene organische und anorganische Säuren wurden zur Reduktion oder Entfernung von CBC-C₅ bei Raumtemperatur in organischem Lösungsmittel in Konzentrationen von 1 Vol-% verwendet.

| Nr. | Protische Säure | Flächenprozent CBC-C₅ nach 2h |
|---|---|---|
| 1 | H₂SO₄ | 0, jedoch vollständige Umwandlung d9-THC in d8-THC |
| 2 | HCOOH | 2,1 |
| 3 | CF₃COOH | 1, 1 |
| 4 | H₃PO₄ | 2,2 |

### Beispiele Reaktionsbedingungen:

Es wurden verschiedene Konzentrationen und Temperaturbedingungen getestet, um die Reduktion oder Entfernung von CBC-C₅ weiter zu optimieren, ohne dass zusätzliches CBN und andere Abbauprodukte entstehen.

| Nr. | c(TFA-Vol-%) | c(Extrakt-mg/ml) | T (°C) | Flächenprozent CBC-C₅ nach 2h |
|---|---|---|---|---|
| 1 | 0,2 | 25 | 23 | 2,2 |
| 2 | 0,4 | 25 | 23 | 2,0 |
| 3 | 0, 6 | 25 | 23 | 1,5 |
| 4 | 1,0 | 25 | 23 | 1, 1 |
| 5 | 1,0 | 15 | 23 | 1, 1 |
| 6 | 1,0 | 40 | 23 | 1,0 |
| 7 | 1,0 | 75 | 23 | 1,7 |
| 8 | 1,0 | 100 | 23 | 2,1 |
| 9 | 1,0 | 40 | 30 | 1,0 |
| 10 | 1,0 | 40 | 50 | 1,2 |

### Zusammenfassung der Erfindung:

Die erfindungsgemäße chemische Reaktion/Umwandlung und chromatographische Reinigung bietet ein neues Verfahren zur Herstellung von reinem THC (pharmazeutischer Wirkstoff API) mit der erforderlichen Reinheit gemäß Pharmakopöe (USP The US Pharmacopeia 42 NF37 oder DAC Deutscher Arzneimittelcodex 2012-2 oder Ph.Eur. 11.5).

Die Verwendung ausgewählter Säuren und Reaktionsbedingungen bietet eine Kontrolle über die anderen Cannabinoid-Nebenprodukte des Extrakts und optimiert das Profil dieser Produkte für die anschließende Reinigung durch CPC, wobei bekanntlich CBC nicht vollständig von THC getrennt werden kann. Daher kann auf weitere kombinierte Reinigungstechniken verzichtet werden.

## Patentansprüche

1. Verfahren zum Herstellen von Cannabinoiden aus einem Cannabis Extrakt, aufweisend folgende Schritte:
i.) Trocknen eines Cannabis Extrakts,
ii.) Aufnehmen von i.) in mindestens einem unpolaren aprotischen Lösungsmittel enthaltend mindestens eine protische Säure, wobei das Verhältnis von protischer Säure zum Extrakt im unpolaren aprotischen Lösungsmittel 0,1 - 3,0 Vol % : 5 - 250 mg/ml beträgt,
iii.) Durchführen einer Flüssig-flüssig-Verteilungschromatographie von ii.) unter Verwendung eines mindestens zweiphasigen Lösungsmittelsystems, wobei eine erste Phase durch Zentrifugalkraft stationär gehalten wird und eine zweite nicht mischbare flüssige Phase,
iv.) Isolieren der Cannabinoide aus iii.).

2. Verfahren zum Herstellen von Cannabinoiden aus einem Cannabis Extrakt nach Anspruch 1, wobei das Cannabinoid Dronabinol oder CBD ist.

3. Verfahren zum Herstellen von Cannabinoiden aus einem Cannabis Extrakt nach einem der vorstehenden Ansprüche, wobei das mindestens eine unpolare aprotische Lösungsmittel gemäß ii.) eine Dielektrizitätskonstante ε bis 15, insbesondere bis 10 bei Normalbedingungen aufweist, insbesondere Alkane, Cycloalkane, Hexan, Heptan, Cyclohexan.

4. Verfahren zum Herstellen von Cannabinoiden aus einem Cannabis Extrakt nach einem der vorstehenden Ansprüche, wobei die protische Säure gemäß ii.) ausgewählt ist aus ein oder mehreren Mineralsäuren, organischen Säuren, insbesondere Salzsäure, Schwefelsäure, Phosphorsäure, Ameisensäure, Essigsäure oder deren halogenierter Varianten.

5. Verfahren zum Herstellen von Cannabinoiden aus einem Cannabis Extrakt nach einem der vorstehenden Ansprüche, wobei vor Schritt iii.) ein Schritt ii*) erfolgt, und zwar Zugabe mindestens eines protischen Lösungsmittels.

6. Verfahren zum Herstellen von Cannabinoiden aus einem Cannabis Extrakt nach Anspruch 5, wobei das protische Lösungsmittel eine Dielektrizitätskonstante ε mehr als 15, insbesondere mehr als 20 bei Normalbedingungen aufweist, insbesondere Alkohole, Methanol, Ethanol.

7. Verfahren zum Herstellen von Cannabinoiden aus einem Cannabis Extrakt nach einem der vorstehenden Ansprüche, wobei in Schritt ii.) die Temperatur 15 - 70, insbesondere 20 - 50 Grad Celsius beträgt, und optional je höher die Volumenkonzentration im Verhältnis gewählt wird, desto niedriger kann die Temperatur gewählt sein.

8. Verfahren zum Herstellen von Cannabinoiden aus einem Cannabis Extrakt nach einem der vorstehenden Ansprüche, wobei in Schritt ii.) die Reaktionszeit 0,1h - 4h insbesondere 0,5h - 3h beträgt, und optional je höher die Temperatur gewählt wird, desto niedriger kann die Reaktionszeit gewählt sein.

9. Verfahren zum Herstellen von Cannabinoiden aus einem Cannabis Extrakt nach einem der vorstehenden Ansprüche, wobei die erste oder zweite Phase gemäß iii.) ausgewählt sind aus n-Hexan, n-Heptan, Cyclo-Hexan, n-Propanol, Chloroform, Ethylacetat, Butylmethylether, Acetonitril, Methanol, Ethanol, Wasser, n-Butanol, Dichlormethan, Tetrahydrofuran, Isooctan, insbesondere eine Mischung davon.
